# EUROPÄISCHE PATENTANMELDUNG

(11) **EP 2 314 253 A1**
(43) Veröffentlichungstag der Anmeldung: **27.04.2011**
(21) Anmeldenummer: 10188212.4
(22) Anmeldetag: 20.10.2010
(51) Int. Cl.: A61C 19/06

(54) **Vorrichtung und Verfahren zur kosmetischen Behandlung von verfärbten Zähnen**

(30) Priorität: 21.10.2009 DE 102009050440
(71) Anmelder: Asclepion Laser Technologies GmbH, 07747 Jena (DE)
(72) Erfinder: Brückner, Daniel, 07745, Jena (DE); Elbrecht, Jens, 07751, Jena (DE); Streit, Ingolf, 07751, Rothenstein (DE)
(74) Vertreter: Geyer, Fehners & Partner

(57) **Zusammenfassung**

Die Erfindung betrifft eine Vorrichtung, mit der Zähne im Mund einer Person bei der Zahnbleichung mittels eines photochemisch aktivierbaren Bleichgels bestrahlt werden. Die Vorrichtung umfaßt Mittel zur Erzeugung von Licht zur Bestrahlung der Zähne, Mittel zur Kühlung der Lichterzeugungsmittel, Lichtverteilungsmittel, durch die das Licht zur gleichzeitigen Bestrahlung einer Vielzahl der Zähne abstrahlbar ist, Mittel zur Steuerung der auf die Zähne gerichteten Lichtdosis sowie Adaptierungsmittel zur Positionierung der Vorrichtung relativ zu den Zähnen.

Die Lichterzeugungsmittel umfassen mindestens ein Array (7) von Licht emittierenden Hochleistungsdioden, die zur Abstrahlung von Licht in Abstrahlwellenlängenbereichen mit einer Halbwertsbreite von jeweils bis zu 100 nm ausgestaltet sind, wobei sich die Abstrahlwellenlängenbereiche mit einem Absorptionswellenlängenbereich mit Wellenlängen, in denen das Bleichgel aktivierbar ist, überlappen. Die Lichtverteilungsmittel umfassen eine Lichtaustrittsfläche (11), von der das Licht auf die Zähne gelenkt wird. Die Lichtaustrittsfläche (11) weist eine Krümmung auf, die im wesentlichen der Krümmung des Gebisses folgt, so daß die Lichtaustrittsfläche (11) zu den Zähnen im Mittel den gleichen Abstand aufweist.

Die Erfindung betrifft auch ein Verfahren zur Bleichung von Zähnen, bei dem insbesondere die genannte Vorrichtung verwendet werden kann.

## Beschreibung

### Gebiet der Erfindung

Die Erfindung bezieht sich auf eine Vorrichtung, mit der Zähne im Mund einer Person bei der Zahnbleichung mittels eines auf die Zähne aufgetragenen, photothermisch aktivierbaren Bleichgels bestrahlt werden. Eine solche Vorrichtung umfaßt Lichterzeugungsmittel zur Erzeugung von Licht zur Bestrahlung der Zähne, Kühlmittel zur Kühlung der Lichterzeugungsmittel, Lichtverteilungsmittel, durch die das von den Lichterzeugungsmitteln erzeugte Licht in einem definierten Bereich zur gleichzeitigen Bestrahlung einer Vielzahl der Zähne abstrahlbar ist, Mittel zur Steuerung der auf die Zähne gerichteten Lichtdosis sowie Adaptierungsmittel zur Positionierung der Vorrichtung relativ zu den Zähnen im Mund. Die Erfindung betrifft auch ein Verfahren zur kosmetischen Bleichung von Zähnen.

### Stand der Technik

Im Stand der Technik ist eine Reihe von Vorrichtungen und Verfahren zur Bleichung von Zähnen beschrieben, solche für Heimanwender und solche, die professionell in der Regel von Zahnärzten durchgeführt werden. Die professionelle Zahnbleichung macht sich die bleichende Wirkung von Peroxid-Verbindungen oder anderen bleichenden Substanzen wie Perboraten zunutze. Die zunächst inaktive Substanz wird mit der zu bleichenden Struktur, der aus dem Mund nach außen weisenden Oberfläche eines Zahn in Kontakt gebracht und danach aktiviert. Solche Substanzen, die zur Bleichung verwendet werden können, sind beispielsweise in der US 6,387,353 beschrieben, in der WO 02/22097, oder auch in der US 6,846,182 B1. Die Aktivierung erfolgt dabei durch Wärme, die entweder direkt - beispielsweise durch Heizwiderstände - erzeugt werden kann, oder indirekt mittels eines hochfrequenten Wechselfeldes oder Licht, aber auch durch Kombination dieser Verfahren. Beispiele für indirekte Erwärmung sind beispielsweise in der US 7,320,595 - hier wird die Erwärmung mittels elektrischer, elektrophoretischer oder elektromagnetischer Wechselfelder erreicht - oder in der US 2003/0157456 A1 - hier wird Licht verwendet - beschrieben.

Die Wirkungsweise solcher peroxidbasierter Aufheller beruht auf der Oxidation verfärbter organischer Komponenten, die sich im Laufe der Zeit in oder auf den Zähnen gebildet oder abgelagert haben. Die Verfärbungen stammen entweder von Chromogenen in Nahrungsmittel-Komponenten und/oder Tabak, oder auch von funktionslosen Chromogenen, die sich mit zunehmendem Lebensalter in der organischen Matrix vom Zahnschmelz bzw. Dentin gebildet oder darin abgelagert haben. Diese unerwünschten Farbänderungen entstehen aufgrund der Absorption von Licht an ungesättigten chemischen Donorbindungen der Chromogene. Diese ungesättigten C=C-Elektronen-Donorbindungen sind gegenüber Peroxiden äußerst instabil und zerfallen unter Zersetzung, wobei sich Alkohole, Carboxylsäuren und unter extremen Bedingungen auch Kohlendioxid und Wasser bilden können. Dabei ist es wichtig zu wissen, daß diese Reaktionsmechanismen von Peroxiden per se in keinem Falle zur Schädigung - hier wären in erster Linie Demineralisationen und Ätzung zu nennen - von Schmelz bzw. Dentin führen. Ganz im Gegenteil sind Peroxide gegenüber Hydroxylapatit, der mineralischen Komponente von Knochen und Zähnen, vollkommen inert.

Wasserstoffperoxid in der chemischen Formel H₂O₂ kann in HO-, O₂-, O₂O-, HOO⁻, HO₂O-Radikale oder -Ionen zerfallen. Diese Zerfallsprodukte von Wassestoffperoxid stellen die aktive Bleichsubstanz dar. Diese wirkt oxidierend auf die Farbstoffe im Zahn und bewirkt dadurch unter Aufspaltung dieser Farbstoffe eine Aufhellung. H₂O₂ ist eine schwache Säure, die mehr freie Sauerstoffradikale (OO) freisetzt als Perhydroxylradikale (HO₂O), bei pH-Werten von mehr als 9,5 setzt sie dagegen mehr Perhydroxyl- als Sauerstoffradikale frei. Das Perhydroxyl-Radikal ist stärker wirksam als das Sauerstoffradikal, die Sauerstofffreisetzung kann durch Hitze, bei Anwesenheit von geeigneten Katalysatoren durch Licht oder Erhöhung des pH-Wertes beschleunigt werden.

Dabei hat die photothermische Erwärmung durch Aktivierung mit Licht Vorteile gegenüber photothermischer Erregung direkt mit Wärme, da die Wärme bei der ersten Methode nur im Gel erzeugt wird. Bei zu hoher Temperatur im Zahn wird dieser nämlich unsensibel gegenüber weiteren Bleichungsbehandlungen.

Neben der Verwendung von Bleichmittel sind natürlich auch mechanische Verfahren wie die Abtragung von Material mittels Abrasion oder Poliermitteln bekannt sowie Ultraschallbehandlungen, derzeit jedoch ist die Methode der Bleichung mittels eines Gels, welches Wasserstoffperoxid verwendet, im professionellen Bereich die erste Wahl. Dazu ist in das Gel zusätzlich zu dem H₂O₂ auch ein Farbstoff eingebracht, der als Ziel für die Lichtabsorption dient. Durch die Absorption der Lichtenergie wird das Bleichgel erwärmt und es kommt zur Aktivierung des Wasserstoffperoxids im Gel. Ist das Gel beispielsweise mit einem roten Farbstoff versetzt, so bedeutet dies, das die Absorption von Licht mit Wellenlängen der Komplementärfarbe, also Grün, besonders hoch ist. In den gängigen Verfahren und Anordnungen werden zur Erzeugung des Lichtes Laser, einzelne lichtemittierende Dioden (LED, Leuchtdioden), UV- und Weißlichtlampen verwendet. Diese Lichtquellen weisen jedoch allesamt einen oder mehrere Nachteile auf, die das Bleichen ineffizient und aufwendig werden läßt.

So wird in der WO 02/22097 ein Verfahren beschrieben, bei dem zum einen ein mit einem Farbstoff wie beispielsweise Rhodamin versetztes Gel verwendet wird, zum anderen ein Laser, mit dem der Farbstoff bestrahlt wird, so daß er dessen Licht absorbiert und in Wärme umwandelt. Laserwellenlängen und Farbstoff werden so gewählt, daß das Laserlicht von dem Farbstoff absorbiert werden kann. Beispielsweise kann eine Laserwellenlänge von 532 nm verwendet werden, diese liegt dann in dem Bereich, in dem Rhodamin Licht absorbiert. Das Laserlicht ist sehr schmalbandig, die maximale Absorption bei Rhodamin findet jedoch bei einer Wellenlänge von 555 nm statt, so daß die Absorption hier auch nicht besonders effizient ist. Da Laserlichtquellen sehr teuer sind, wird in der Regel ein Laser mit geringerer Leistung verwendet, so daß die Zähne dann einzeln nacheinander behandelt werden müssen. Dazu werden sie mit einer Lichtleitfaser beleuchtet. Aufgrund des stark divergenten Strahls, der aus dem Ende der Lichtleitfaser tritt, zeigt die Flächenleistungsdichte, die eingetragen wird, eine hohe Abhängigkeit vom Abstand des Faserendes zum Zahn. Die Strahlverteilung und die Flächenleistungsdichtedichte hängen außerdem noch von der Biegung der Fasern ab, so daß das Verfahren nicht ohne weiteres reproduzierbar ist. Eine gleichmäßige Behandlung mit definierten Flächenleistungsdichten ist auf diese Weise unmöglich, so daß während der ganzen Dauer der Behandlung die Anwesenheit des Behandlers notwendig ist. Da die Behandlung Zahn für Zahn erfolgt, besteht außerdem die Möglichkeit, daß bereits aufgetragenes, jedoch inaktives Bleichmittel vor der Aktivierung in einen noch nicht behandelten Zahn diffundiert und wirkungslos wird. Eine Erhöhung der Laserleistung andererseits, mit der die gleichzeitige Bestrahlung vieler Zähne erreicht werden könnte, bedarf hoher Laserleistung, was zum einen die verwendeten Geräte sehr viel teuerer machen würde, zum anderen aber auch die Gefahr von bleibenden Schäden, beispielsweise an den Augen bei unsachgemäßer Behandlung wesentlich erhöht.

In der US 2007/0015112 A1 wird eine Vorrichtung zum Bleichen von Zähnen beschrieben, bei der die Erwärmung des Gels zur Aktivierung über Wärme und/oder über Licht erfolgen kann. Die Vorrichtung ist nach Art einer Zahnbürste ausgestaltet, so daß sie im Prinzip auch für den Heimverbrauch verwendet werden kann, wobei jedoch das Bleichmittel in der Regel nur in niedriger Konzentration verwendet werden darf. Für den professionellen Gebrauch ist diese Vorrichtung nicht geeignet.

In der US 7,320,595 B2 wird eine Anordnung zur Zahnbleichung beschrieben, bei der eine der Krümmung der Zähne folgende Vorrichtung in den Mund eingesetzt wird. Die Erwärmung des Gels erfolgt zum einen über Elektrophorese, zum anderen ist auch die Verwendung von einigen wenigen LEDs vorgesehen, die zusätzlich in das Gel Licht eintragen, so daß dieses erwärmt wird.

Auch in der US 2005/002613 A2 sowie in der US 2004/0110111 A1 sind Vorrichtungen für den Heimgebrauch beschrieben, die in den Mund der zu behandelnden Person eingesetzt werden. Eine solche Vorrichtung weist beispielsweise eine Zellstruktur auf, in der das Bleichmittel enthalten ist. Gleichzeitig enthält die Vorrichtung, die zur einmaligen Verwendung gedacht ist, eine einmal zu aktivierende phosphoreszierende Lichtquelle, die dazu dient, die bleichenden Bestandteile zu aktivieren. Die Lichtquelle wird vor dem Einsetzen der Vorrichtung in den Mund aktiviert.

Auf dem Markt sind weiterhin verschiedene Einrichtungen bekannt, die zur Beleuchtung beispielsweise UV-Lampen, Weißlichtlampen, oder gewöhnliche LEDs verwenden. So wird beispielsweise von der Firma Britesmile eine Anordnung vertrieben, bei der 400 bis 500 Weißlicht-LEDs entlang eines Bogens angeordnet sind, wobei aus dem abgestrahlten Licht jedoch nur ein Blauanteil im Bereich zwischen 400 und 420 nm herausgefiltert wird, der dazu benötigt wird, das vorher aufgebrachte Gel zu aktivieren. Die Behandlung dauert eine Stunde, das Gel muß dabei mehrfach aufgetragen werden. Die Anordnung ist aufgrund der hohen Anzahl von LEDs sehr groß, muß also außerhalb des Mundes plaziert werden. Das Licht der LEDs ist zudem sehr hell, so daß die zu behandelnde Person einen Augenschutz tragen muß. Aufgrund der hohen Anzahl von LEDs die notwendig sind, um überhaupt die erforderliche Leistungsdichte zur Aktivierung des Gels zu erzielen, wird außerdem eine aufwendige Kühlung erforderlich, die hier mittels Luftkühlung realisiert wird. Aufgrund der divergenten Abstrahlung des Lichtes ist die Leistungsdichte in der Fläche darüber hinaus stark abstandsabhängig, so daß die Behandlung in der Regel nicht genau reproduzierbar ist, da bereits wenige Millimeter bei der Variation des Abstandes der Beleuchtungseinrichtung zu den Zähnen zu wesentlich unterschiedlichen Ergebnissen führen können.

Effektiver ist die Anwendung von blauen LEDs, beispielsweise in einer Vorrichtung der Firma Whitesmile. Hier sind in einer Lampe, die ebenfalls außerhalb des Mundes der zu behandelnden Person angeordnet wird, mehrere LEDs in einem Bogen angeordnet, von denen ein Teil Licht in einem blauen Wellenlängenbereich, ein anderer Teil im cyanen Wellenlängenbereich, insgesamt also in einem Wellenlängenbereich zwischen 480 und 510 nm abstrahlt. Bei einer Gesamtleistung von 30 Watt beträgt die Flächenleistungsdichte 2500 mW/cm². Auch diese Vorrichtung wird außerhalb des Mundes angeordnet, die zu behandelnde Person muß einen Schutz tragen. Auch hier geht also ein hoher Teil an Leistung verloren, zudem ist die schon beschriebene Abstandsabhängigkeit vorhanden. Auch hier ist eine aufwendige Luftkühlung verbaut. Aufgrund der Anordnung außerhalb des Mundes und der Abhängigkeit der Leistungsdichte vom Abstand, verursacht durch die breiten Abstrahlwinkel der LEDs, ist auch hier eine Reproduzierbarkeit der Ergebnisse nicht gewährleistet, so daß die zu behandelnden Personen dauernd überwacht werden müssen.

### Beschreibung der Erfindung

Der Erfindung liegt daher die Aufgabe zugrunde, eine Vorrichtung der eingangs beschriebenen Art gegenüber dem Stand der Technik zu verbessern, insbesondere dahingehend, daß mit der Anordnung reproduzierbare Ergebnisse erzielt werden können, grundsätzlich also eine unbeaufsichtigte Behandlung möglich ist. Die Vorrichtung soll dabei außerdem so klein sein, daß sie direkt im Mund plaziert werden kann, wobei das Auftreten von störenden Geräuschen und Abluftströmen während der Behandlung möglichst unterdrückt werden soll.

Diese Aufgabe wird bei einer Vorrichtung der eingangs beschriebenen Art dadurch gelöst, daß die Lichterzeugungsmittel mindestens ein Array von in einer Abstrahlfläche angeordneten, licht emittierenden Hochleistungsdioden (auch bezeichnet als Hochleistungsleuchtdioden, H-LED) umfassen, wobei die Hochleistungsdioden zur Abstrahlung von Licht in Abstrahlwellenlängenbereichen mit einer Halbwertsbreite von jeweils bis zu 100 nm ausgestaltet sind und sich die Abstrahlwellenlängenbereiche mit einem Absorptionswellenlängenbereich mit Wellenlängen, in denen das Bleichgel aktivierbar ist, überlappen, und die Lichtverteilungsmittel eine Lichtaustrittsfläche umfassen, von der das Licht auf die Zähne gelenkt wird, wobei die Lichtaustrittsfläche eine Krümmung aufweist, die im wesentlichen der Krümmung des Gebisses folgt, so daß bei in den Mund eingebrachter Vorrichtung die Lichtaustrittsfläche zu den zu bestrahlenden Zähnen im Mittel den gleichen Abstand aufweist. Mit Abstand ist die kürzeste Entfernung zwischen der Oberfläche eines Zahns und der Lichtaustrittsfläche gemeint.

Die erfindungsgemäße Vorrichtung bietet gleich mehrere Vorteile. Da als Lichtquelle mindestens ein Array von Hochleistungs-Leuchtdioden, beispielsweise 4x4, 5x5 oder 6x6 Leuchtdioden, die in einer Abstrahlfläche anordnet sind, verwendet werden, läßt sich relativ problemlos die notwendige geforderte Flächenleistungsdichte von etwa 200 mW/cm² erreichen, die zur Aktivierung des Bleichgels notwendig ist. Da jede der Hochleistungs-Leuchtdioden selbst eine hohe Leistung von 2,5 Watt - gegenüber etwa 50 mW von normalen Leuchtdioden - aufweist, werden zur Erzielung der Leistungsdichte nur wenige Leuchtdioden benötigt. Die Halbwertsbreite der Abstrahlwellenlängenbereiche der Dioden, die von Diode zu Diode aufgrund von Fertigungstoleranzen um bis zu etwa 10 nm in ihrer Lage leicht variieren können, weisen dabei bei einer Halbwertsbreite von jeweils bis zu 100 nm eine eher schmalbandige Charakteristik auf. Die Abstrahlwellenlängenbereiche werden außerdem so gewählt, daß sie sich mit einem Absorptionswellenlängenbereich mit Wellenlängen, in denen das Bleichgel aktivierbar ist, überlappen. Aufgrund dieser beiden Einstellungen ist eine sehr effiziente Nutzung des von den Leuchtdioden abgestrahlten Lichts für die Aktivierung des Bleichgels möglich, da zum einen nur ein kleiner Wellenlängenbereich verwendet wird, dessen Lage zum anderen so gewählt wird, daß ein großer Teil des in diesem Wellenlängenbereich abgestrahlten Lichts auch absorbiert wird. Dazu kann das Bleichgel beispielsweise auch mit einem Farbstoff markiert sein, dessen Farbe entsprechend zur Zentralwellenlänge - der Wellenlänge, bei der die Intensität des abgestrahlten Lichtes maximal ist - im wesentlichen, d.h. in einem Bereich von wenigen 10 nm, komplementär ist. Wird also beispielsweise grünes Licht zur Bestrahlung verwendet, so markiert man das Bleichgel vorzugsweise mit einem roten Farbstoff.

Während im Stand der Technik bei außerhalb des Mundes angeordneten Vorrichtungen Flächenleistungsdichten von bis zum 2500 mW/cm² erreicht werden müssen, da ein großer Anteil des Lichtes nicht auf das zu aktivierende Bleichgel trifft, sondern in die Umgebung gestreut wird und zudem die Entfernung zu den Zähnen größer ist, kommt die erfindungsgemäße Vorrichtung bereits mit Flächenleistungsdichten von 200 mW/cm², also etwa einem Zehntel davon, aus. Der Grund dafür liegt zum einen in der nahen Anordnung relativ zu den Zähnen, d.h. in der Ausgestaltung der Lichtaustrittsfläche, auf die das von den Lichterzeugungsmitteln erzeugte Licht gelenkt wird und durch diese auf die Zähne abgestrahlt wird. Die Krümmung der Lichtaustrittsfläche folgt der Krümmung des Gebisses, d.h. daß bei in den Mund eingebrachter Vorrichtung die Lichtaustrittsfläche zu den zu bestrahlenden Zähnen in Mittel den gleichen Abstand aufweist, genauer gesagt, zu den Oberflächen der zu bestrahlenden Zähne, die also nach außen weisen und bei geöffnetem Mund für Dritte sichtbar sind. Im Gegensatz zu den üblichen Bestrahlungsvorrichtungen für den professionellen Einsatz - und nur für diesen - ist also vorgesehen, daß die zu behandelnde Person die Bestrahlungsvorrichtung mindestens teilweise in den Mund nimmt. Dies ist nur möglich, da schmalbandig abstrahlende Hochleistungs-Leuchtdioden verwendet werden, deren Abstrahlcharakteristiken an den oder die Farbstoffe, mit denen das Bleichgel versetzt ist, angepaßt sind. Da die Lichtaustrittsfläche auch in ihrer Größe an das Gebiß angepaßt werden kann, ist die Lichtnutzung sehr effizient, es findet keine zusätzliche Bestrahlung von nicht zu behandelnden Bereichen statt, die Wärmeentwicklung ist dementsprechend gering und die zu behandelnder Person wird nicht geblendet, braucht also keinen Augenschutz.

Die Hochleistungs-LEDs selbst andererseits weisen eine starke Wärmeentwicklung auf, so daß hier eine effiziente Kühlung notwendig ist, um insbesondere auch eine kompakte Bauweise gewährleisten zu können, die das Einbringen in den Mund möglich macht. Die Wärme der LED wird dabei jedoch vorteilhaft außerhalb des Mundes erzeugt. Während im Stand der Technik für die Kühlung von LED - bei den für den Heimgebrauch verwendeten Geräten ist eine Kühlung in der Regel nicht notwendig oder vorgesehen - üblicherweise Peltier-Elemente und / oder Luftkühlungsvorrichtungen verwendet werden, ist erfindungsgemäß bei den Kühlungsmitteln bevorzugt eine Wasserkühlung mit mindestens einer Zu- und mindestens einer Ableitung vorgesehen. Der Durchfluß des Wassers kann dabei beispielsweise mittels eines Temperatursensors überwacht werden. Zwar erfordert diese Art von Kühlung zusätzliche Maßnahmen wie die genannten Zu- und Ableitungen für Wasser, die ebenfalls in die Vorrichtung integriert werden müssen; da diese Art der Kühlung jedoch sehr effizient und leise ist, ist dieser Mehraufwand durchaus zu vertreten. Insbesondere wird die zu behandelnde Person während der Dauer der Behandlung nicht durch eine Lärmquelle in unmittelbarer Nähe belästigt. Demgegenüber werden die im Stand der Technik beschriebenen Vorrichtungen nur über Luftkühler, umfassend in der Regel Ventilatoren, gekühlt, die sich einfach über die sowieso notwendige Stromversorgung betreiben lassen.

In einer bevorzugten Ausgestaltung der Erfindung beträgt die Halbwertsbreite der Abstrahlwellenlängenbereiche 30 nm bis 50 nm. Bei entsprechender Anpassung der Überlappung der Abstrahlwellenlängebereiche mit dem Absorptionswellenlängenbereich wird die Effizienz weiter gesteigert. Alternativ oder ergänzend können die Charakteristika der H-LED auch so eingestellt werden, daß die Zentralwellenlängen der Abstrahlwellenlängenbereiche in einem Bereich von 30 nm um eine Wellenlänge, bei der die Aktivierung des Bleichmittels maximal ist, liegen. Auch dies führt zur Effizienzsteigerung.

In einer besonders bevorzugten Ausgestaltung der Erfindung umfassen die Lichterzeugungsmittel drei Arrays von Licht emittierenden Hochleistungsdioden, wobei die Abstrahlfläche jedes Arrays zu den Abstrahlflächen der jeweils anderen Arrays um einen in bezug auf das jeweilige andere Array spezifischen Kippwinkel gekippt angeordnet ist. Im Zusammenspiel mit den Lichtverteilungsmitteln und insbesondere der Lichtaustrittsfläche läßt sich auf diese Weise die Intensitätsverteilung des durch die Lichtaustrittsfläche abgestrahlten Lichtes an verschiedene Bedürfnisse anpassen. Die geometrischen Schwerpunkte der Arrays liegen dabei bevorzugt auf einer Linie, das mittlere der Arrays kann dann beispielsweise für die Beleuchtung des Frontzahnbereichs verwendet werden. Die beiden rechts und links davon positionierten Arrays sind dann in bezug auf das mittlere Array beispielsweise um einen Kippwinkel von gleichem Betrag, aber mit unterschiedlichem Vorzeichen gekippt. Im Zusammenspiel mit den noch zu beschreibenden Lichtverteilungsmitteln und in Abhängigkeit davon werden dann bei flachen Kippwinkeln die Lichtstrahlen beispielsweise eher auf den Seitenzahnbereich, bei steilen Kippwinkeln auf den Vorder- oder Eckzahnbereich gelenkt. Auch andere Anordnungen, bei denen bei steilen Kippwinkeln nach außen das Licht auf den Seitenzahnbereich gelenkt wird, sind denkbare Ausgestaltungen.

Während das mittlere Array den Schneidezähnen frontal gegenüberliegt, sind die beiden äußeren Arrays - die Linie, auf der die Arrays liegen, liegt in der Bißebene - um besagten Kippwinkel nach außen gerichtet, das Licht wird durch die Lichtverteilungsmittel entsprechend umgelenkt. Dabei ist es durchaus möglich, Mittel vorzusehen, mit denen die Kippwinkel variiert werden können, um eine Anpassung an verschiedene Bedürfnisse durch eine Variation der Intensitätsverteilung zu erreichen.

Die Lichtverteilungsmittel umfassen bevorzugt entsprechend der Anzahl von Arrays Lichtleitkanäle mit Lichteintrittsflächen, die bevorzugt parallel zu den Abstrahlflächen angeordnet sind, um das von den LED abgestrahlte Licht möglichst vollständig aufzunehmen. Die Lichteintrittsflächen sind dabei aus demselben Grund so nah wie möglich an den Abstrahlflächen angeordnet. Aus dem gleichen Grund weisen die Lichteintrittsflächen auch Durchmesser in der Größenordnung der LED-Arrays auf, sofern sie rund ausgestaltet sind. Auch Ausgestaltungen der Kanäle mit Kanten, also in eckiger Form, sind denkbar, für eine möglichst verlustarme Lichtübertragung und Verteilung sind jedoch runde Kanäle zu bevorzugen. Die Lichtleitkanäle können beispielsweise aus einem Material gestaltet sein, welches für die Abstrahlwellenlängenbereiche überwiegend transparent ist. Im einfachsten Fall sind sie dann als einfache Glasstäbe ausgestaltet. Diese Glasstäbe können dann beispielsweise auch mit einer Reflexionsschicht versehen sein, die verhindert, daß Licht von den Rändern der Kanäle nach außen tritt. Auf die Glasstäbe kann aber auch verzichtet werden, im einfachsten Falle bestehen die Lichtleitkanäle dann aus hohlen Röhrchen, die dann bevorzugt mit der schon erwähnten Reflexionsschicht mit Wirkung nach innen versehen sind.

An die Lichtleitkanäle an schließt sich ein Lichtverteilungsraum, der durch eine oder mehrere Begrenzungsflächen zu den Seiten, wohin kein Licht abgestrahlt werden soll, und durch die Lichtaustrittsfläche, durch die das Licht austreten soll und die zu den Lichtleitkanälen beabstandet angeordnet ist, begrenzt bzw. abgeschlossen wird. Die Lichtaustrittsfläche liegt also in der Regel den Lichtleitkanälen gegenüber, diese bilden ebenfalls eine Begrenzung des Lichtverteilungsraums. Auch bei der Ausgestaltung der Begrenzungsflächen gilt es, darauf zu achten, daß diese nicht mehr Kanten als notwendig aufweisen, um die Lichtverluste möglichst gering zu halten. Um das Licht auf die Lichtaustrittfläche umzulenken sind die Begrenzungsflächen vorteilhaft nach innen in den Lichtverteilungsraum reflektierend ausgestaltet. In den Lichtverteilungsraum über die Lichtleitkanäle einfallendes Licht kann diesen also - abgesehen von Photonen, die in die Lichtleitkanäle zurückgestreut werden - nur über die Lichtaustrittsfläche verlassen. Die reflektierenden Begrenzungsflächen des Lichtverteilers sind vorzugsweise so gestaltet, daß durch diese das von den Zahnoberflächen reflektierte Licht zumindest teilweise in Richtung der Zahnreihe zurück gelenkt wird. Dadurch kann dieses Licht für die Aktivierung des Bleachingprozesses wieder nutzbar gemacht werden. Bei nicht reflektierend ausgestatteten Begrenzungsflächen wird zwar ebenfalls noch ein großer Teil über die Lichtaustrittsfläche nach außen gelenkt, ein kleiner Teil würde jedoch entweder durch die Begrenzungsflächen nach außen treten, oder, falls diese mit einer deckenden Schicht versehen sind, von dieser beispielsweise absorbiert werden. Die Ausgestaltung des Lichtverteilungsraumes und der Begrenzungsflächen erfolgt dabei insbesondere auch in Abstimmung mit den Kippwinkeln der Arrays von H-LED, bzw. mit der Lage der Lichtleitkanäle. Wird das Licht von den Arrays über die Lichtleitkanäle nach außen gelenkt, so kann durch eine entsprechend gekrümmte Ausgestaltung der Begrenzungsflächen das Licht auf die betreffenden Zahnpartien umgeleitet werden.

Dabei kann der Lichtverteilungsraum beispielsweise mit einem für die Abstrahlwellenlängenbereiche transparenten Material ausgefüllt ausgestaltet sein. Dies kann beispielsweise das gleiche Material sein, welches auch für die Lichtleitkanäle verwendet werden kann, so daß hier eine einstückige Fertigung möglich ist. Ein großer Teil des Lichtes wird dann an den Seiten der Begrenzungsflächen, die dem Lichtverteilungsraum zugewandt sind, totalreflektiert. Zur Verbesserung der Lichtausbeute kann das transparente Material an den Begrenzungsflächen aber auch mit einer - nach innen, in den Lichtverteilungsraum hinein - reflektierenden Schicht versehen sein.

In einer alternativen und preiswerter zu fertigenden Ausführung ist der Lichtverteilungsraum allerdings als Hohlraum ausgestaltet. Die Begrenzungsflächen werden dann durch eine entsprechende teilweise Einhausung, beispielsweise in Form von Plastik gebildet, auch hier kann wieder eine reflektierende Beschichtung der Innenseiten, die dem Lichtverteilungsraum zugewandt sind, vorgesehen sein. Dabei muß darauf geachtet werden, daß die Lichtaustrittsfläche weiterhin transparent bleibt. Für die Einhausung kann dann beispielsweise transparenter Kunststoff verwendet werden, der entsprechend teilweise mit einer Reflexionsschicht versehen wird. Denkbar ist auch der völlige Verzicht auf eine materiell ausgestaltete Lichtaustrittsfläche, diese kann auch als diejenige Fläche definiert sein, durch die das Licht den Lichtverteilungsraum verläßt, ohne durch eine Grenzfläche hindurchzutreten. Eine materiell ausgestaltete Lichtaustrittsfläche bietet andererseits aber den Vorteil, daß die dahinterliegenden Bereiche vor Verschmutzung und anderen Fremdeinwirkungen geschützt sind.

Auch wenn der Lichtverteilungsraum als Hohlraum ausgestaltet ist, können die Lichtleitkanäle mit den oben schon erwähnten transparenten Material ausgefüllt sein, sie können aber ebenso auch als Hohlraum ausgestaltet sein.

Mit den Adaptierungsmitteln wird die Vorrichtung relativ zu den Zähnen positioniert. Im Stand der Technik bedeutet dies, daß die Beleuchtungseinrichtung in einem mehr oder weniger festen Abstand vor der zu behandelnden Person aufgestellt wird, bzw. in einer festen Lage vor dem Mund des Patienten arretiert wird, wobei der Kopf der zu bestrahlenden Person in der Regel auch auf einer Auflage fixiert ist. Da die erfindungsgemäße Vorrichtung jedoch teilweise in den Mund eingebracht wird, müssen besondere Adaptierungsmittel vorgesehen werden, die ein Verrutschen der Vorrichtung während der Behandlung verhindern. Bevorzugt weisen die Adaptierungsmittel daher Beißauflagen auf, auf die die Person während der Behandlung beißt, so daß die einmal vorgegebene Position der Vorrichtung im Mund dauerhaft während der Behandlung beibehalten wird. In einer besonders bevorzugten Ausgestaltung weisen die Adaptierungsmittel außerdem Anschlüsse zum Anschluß von Absaugleitungen auf, durch die während der Behandlung Speichel abgesaugt werden kann.

Während bei den im Stand der Technik zur professionellen Zahnbleichung erhältlichen Vorrichtungen die Lichtleistung konstant ist und eine Einstellung der einzutragenden Leistung nur über die Betriebszeit vorgenommen werden kann, weist die erfindungsgemäße Vorrichtung in einer bevorzugten Ausgestaltung Mittel auf, mit denen die Lichtleistung im zeitlichen Mittel über eine Pulsweitenmodulation variabel einstellbar ist. Dies ermöglicht beispielsweise konstante Behandlungszeiten bei unterschiedlichen Lichtleistungen, je nach Anforderung.

In einer besonders bevorzugten Ausgestaltung der Erfindung ist die Vorrichtung modular aus von einem Bediener ohne mechanische Hilfsmittel separier- und zusammenfügbaren Modulen zusammengesetzt. Als praktisch hat sich dabei ein Aufbau aus mindestens drei Modulen erwiesen. Das erste Modul ist ein Grundkörper, der als Handstück ausgestaltet ist und beispielsweise in eine entsprechende Halterung außerhalb des Mundes eingesetzt werden kann. In diesem Grundkörper sind unter anderem die Lichterzeugungsmittel und die Kühlungsmittel integriert. Bei korrekter Verwendung befindet sich der Grundkörper außerhalb des Mundes, so daß die entstehende Wärme auch nicht zu einer unerwünschten Erwärmung der Mundhöhle bzw. der Zähne führen kann. Das zweite Modul ist ein als Aufsatz ausgestalteter Aufsatzkörper, der mindestens einen Teil der Lichtverteilungsmittel umfaßt. So können beispielsweise die Lichtleitkanäle Teil des Grundkörpers sein. Insbesondere der Lichtverteilungsraum mit der Lichtaustrittsfläche ist Teil des Aufsatzkörpers. Auch die Lichtleitkanäle können als Teil des Aufsatzkörpers ausgebildet sein, so daß dieser die Lichtverteilungsmittel vollständig umfaßt. Ein drittes Modul wird durch einen Adapter gebildet, der die Adaptierungsmittel umfaßt, wobei dieses dritte Modul aber auch mit dem Aufsatzkörper fest verbunden ausgestaltet sein kann.

Diese modulare Bauweise hat den Vorteil, daß die in der Herstellung und Wartung aufwendigeren Bauteile, wie Lichterzeugungsmittel und Wasserkühlung alle in den Grundkörper integriert sind und sowohl der Aufsatzkörper als auch der Adapter als Verschleißstücke konzipiert werden können. Dies ist insbesondere insofern vorteilhaft, als nach der Verwendung immer eine Desinfizierung des Gerätes erfolgen muß. Diese kann aber ebenso wie die Behandlung selbst die Lichtaustrittsfläche in ihrer Qualität beieinträchtigen, in dem sie sie beispielsweise mit der Zeit stumpf werden läßt. Nach etwa zehn Behandlungen ist damit zu rechnen, daß hier ein Austausch vorgenommen werden muß. Einem aufwendigen Auseinanderbauen des gesamten Gerätes mit anschließendem Wechsel bestimmter Teile kann so vorgebeugt werden.

Die erfindungsgemäße Vorrichtung verfügt weiterhin über Mittel zur Steuerung der auf die Zähne gerichteten Lichtdosis. So wird die Lichtdosis beispielsweise angepaßt, indem die Lichtleistung im zeitlichen Mittel über eine Pulsweitenmodulation variabel einstellbar ist. Alternativ dazu ist die Lichtdosis durch die Variation der Bestrahlungsdauer einstellbar.

Die Erfindung betrifft auch ein Verfahren zur kosmetischen Bleichung von Zähnen. Bei einem solchen Verfahren wird auf die zu behandelnden Zähne in einem ersten Schritt ein passives, Chromophore enthaltendes und photochemisch aktivierbares Bleichgel aufgebracht. Die Chromophore sind in der Lage, Licht mit Wellenlängen aus einem entsprechenden Absorptionswellenlängenbereich zu absorbieren. Die zu behandelnden Zähne werden dann gleichzeitig und im wesentlichen im gleichen Abstand zu einer Lichtaustrittsfläche, durch die mit von einer Lichtquelle abgestrahltes Licht austritt, beleuchtet, wobei das Licht Wellenlängen aus einem Abstrahlwellenlängenbereich aufweist, dessen Halbwertsbreite nicht größer als 100 nm ist und der sich mit dem Absorptionswellenlängenbereich mindestens teilweise überlappt. Auf diese Weise regt das Licht die Chromophore effizient an, diese wandeln die absorbierte Energie in Wärme um, so daß als Folge auf diese Weise im Bleichgel enthaltendes Wasserstoffperoxid (H₂O₂) aufgespaltet wird. Die Spaltungsprodukte des Wasserstoffperoxids oxidieren die im Zahn enthaltenen Farbstoffe und bleichen somit den Zahn.

Für die Durchführung des Verfahrens kann beispielsweise eine Vorrichtung verwendet werden, wie sie vorangehend beschrieben wurde. Als Lichtquelle werden bevorzugt Hochleistungsleuchtdioden (H-LED) verwendet.

Vor dem Auftragen des Bleichgels kann ein Passivierungsgel auf das Zahnfleisch aufgetragen werden. Außerdem kann auch ein Wangen- und Lippenspreizer in den Mund zu dessen dauerhafter Öffnung während der Behandlung eingebracht werden. Im Anschluß an die Behandlung und an eine Reinigung kann eine weiteres Bleichgel mit einer gegenüber dem zuerst verwendeten Bleichgel niedrigeren Konzentration von Wasserstoffperoxid und einem Fluoranteil auf die Zähne aufgebracht werden, so daß die Zähne bei einer weiteren Bestrahlung versiegelt werden. Während das zuerst verwendete Bleichgel beispielsweise eine Konzentration von 35 % H₂O₂ aufweist, muß die Konzentration des bei der Versiegelung verwendeten Bleichgels nur etwa 10 % betragen.

Nachdem die Chromophore aktiviert bzw. ausgeblichen sind, kann auch kein weiteres Wasserstoffperoxid aufgespalten werden, so daß die Behandlung automatisch zu Ende ist, auch wenn noch Licht abgestrahlt wird. Dieses Licht führt jedoch aufgrund seiner Wellenlänge nicht zu einer Erwärmung der Zähne, welche wiederum eine unerwünschte Passivierung gegen weiteres Bleichen zur Folge haben könnte. Optional kann jedoch die Vorrichtung automatisch abgeschaltet werden, wenn beispielsweise über Sensoren festgestellt wird, daß die Chromophore im Bleichgel ausgeblichen sind. Im Rahmen des erfindungsgemäßen Verfahrens liegt es aber auch, die Aktivierung des Bleichmittels automatisch zu beenden, wenn die Chromophore im Bleichgel ausgeblichen sind.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

### Kurze Beschreibung der Zeichnungen

Nachfolgend wird die Erfindung beispielsweise anhand der beigefügten Zeichnungen, die auch erfindungswesentliche Merkmale offenbaren, noch näher erläutert. Es zeigen:
- Fig.1: eine Gesamtansicht einer Bestrahlungsvorrichtung,
- Fig.2: ein Handstück mit Aufsatz und Adapter,
- Fig.3: Lichterzeugungsmittel im Detail,
- Fig.4: ein Aufsatzstück im Detail und
- Fig.5: einen Adapter im Detail.

### Ausführliche Beschreibung der Zeichnungen

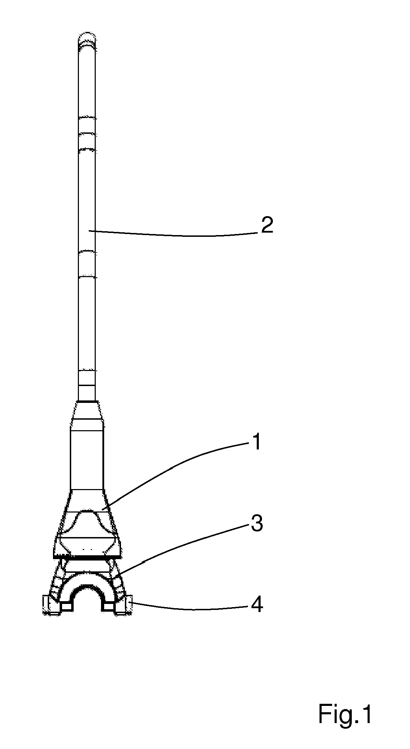

In Fig.1 ist eine Vorrichtung gezeigt, mit der Zähne im Mund einer Person bei der Zahnbleichung mittels eines auf die Zähne aufgetragenen, photochemisch aktivierbaren Bleichgels bestrahlt werden. Die Vorrichtung gliedert sich in mehrere Teile. In einem Handstück 1, mit dem die Vorrichtung von einem Behandler in den Mund einer zu behandelnden Person eingesetzt werden kann, und welches gleichzeitig dazu ausgelegt ist, von einer entsprechend angepaßten Halterung während der Behandlung gehalten zu werden, befinden sich die zentralen technischen Mittel zur Erzeugung von Licht zur Bestrahlung der Zähne sowie die Mittel zur Kühlung der Lichterzeugungsmittel. Von dem Handstück 1 geht in rückwärtiger Richtung - der zu behandelnden Person abgewandt - ein Schlauch 2 ab. Dieser Schlauch enthält neben den elektrischen Leitungen sowie gegebenenfalls Datenleitungen zur Steuerung des Behandlungsprozesses auch die Zu- und Ableitungen der Kühlmittel, die im vorliegenden Beispiel als Wasserkühlung ausgestaltet sind. Die Steuerung des Behandlungsprozesses erfolgt beispielsweise über die auf die Zähne gerichtete Lichtdosis, welche durch die Lichtleistung und die Bestrahlungsdauer bestimmt wird. Auf der anderen Seite des Handstücks 1, der zu behandelnden Person zugewandt, schließt sich ein Aufsatzkörper 3 an. In diesen Aufsatzkörper 3 sind mindestens teilweise Lichtverteilungsmittel integriert, durch die das von den Lichterzeugungsmitteln erzeugte Licht in einem definierten Bereich zur gleichzeitigen Bestrahlung einer Vielzahl der Zähne abstrahlbar ist. Auf diesen Aufsatzkörper 3 wiederum aufgesetzt ist ein Adapter 4, der Adaptierungsmittel bzw. Positionierungsmittel zur Positionierung der Vorrichtung relativ zu den Zähnen umfaßt.

Die Vorrichtung kann modular aufgebaut sein, d.h. daß das Handstück 1, der Aufsatzkörper 3 und der Adapter 4 von einem Bediener ohne mechanische Hilfsmittel separier- und zusammenfügbar sind, gegebenenfalls kann dann auch der Schlauch 2 auf diese Weise vom Handstück 1 getrennt werden. Dies erleichtert insbesondere das Auswechseln des Aufsatzkörpers 3 und des Adapters 4, was beispielsweise erforderlich sein kann, wenn Aufsatzkörper 3 und Adapter 4 für verschiedene Mundgrößen zur Verfügung gestellt werden, oder aber wenn eines der beiden Teile aufgrund von Verschleiß oder Hygienevorschriften ausgewechselt werden muß. Alternativ ist natürlich auch eine Vorrichtung möglich, bei der die Teile nicht nur kraftschlüssig, sondern auch formschlüssig zusammengefügt sind und beispielsweise nur durch das Lösen von Schrauben voneinander getrennt werden können.

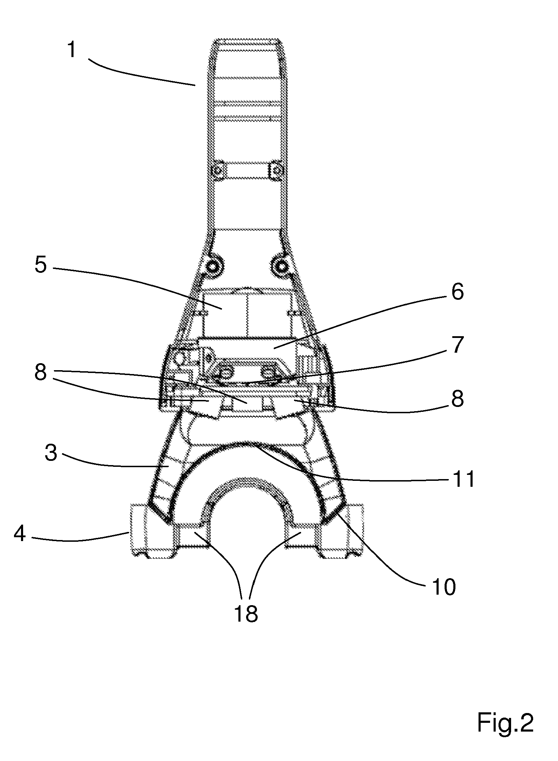

Bei der in Fig.2 gezeigten Detaildarstellung wurde der Schlauch weggelassen und das Handstück 1 geöffnet dargestellt. Die technischen Elemente sind im wesentlichen in dem Bereich angeordnet, wo sich das Handstück 1 verbreitert. Einen Großteil des Platzes nimmt die Wasserkühlung ein, sie umfaßt einen Wärmetauscher 5 und eine LED-Kühlung 6. Die Zu- und Ableitungen für das Wasser wurden nicht dargestellt. Da der Motor, der für den Wasserdurchfluß sorgt, jedoch außerhalb der Vorrichtung in einem externen Gerät, welches von der zu behandelnden Person entfernt angeordnet werden kann, untergebracht ist, handelt es sich bei der Kühlung um eine extrem leise Kühlung, die zudem auch sehr effizient ist und wenig Platz im Vergleich zu Kühlvorrichtungen basierend auf Ventilatoren benötigt, auch wenn der konstruktive Aufwand etwas höher ist. Mit der LED-Kühlung 6 verbunden sind Arrays 7 von in einer Abstrahlfläche angeordneten, lichtemittierenden Hochleistungsdioden. Die Hochleistungsdioden sind zur Abstrahlung von Licht in Abstrahlwellenlängenbereichen mit einer Halbwertsbreite von jeweils bis zu 100 nm ausgestaltet, die Abstrahlwellenlängenbereiche der Leuchtdioden, die aufgrund von Fertigungstoleranzen eine gewisse Schwankungsbreite von bis zu 10 nm aufweisen können, überlappen sich mit einem Absorptionswellenlängenbereich mit Wellenlängen, in denen das Bleichgel aktivierbar ist. Von solchen Arrays 7 mit Hochleistungsleuchtdioden wird mindestens eines benötigt, das Bestrahlungsergebnis und die Lichtverteilung lassen sich jedoch erheblich besser einstellen, wenn beispielsweise drei Arrays verwendet werden.

Die Lichtverteilungsmittel umfassen entsprechend der Anzahl von Arrays 7 Lichtleitkanäle 8 mit Lichteintrittsflächen 9, die bevorzugt parallel zu den Abstrahlflächen angeordnet sind. Die Durchmesser der Lichtleitkanäle 8 entsprechen von der Größenordnung her der Größe der Arrays 7 und sind bevorzugt etwas größer als diese, um auch seitlich von den Arrays aufgrund der Konstruktion der LEDs abgestrahltes Licht noch einzufangen und in einen Lichtverteilungsraum zu leiten.

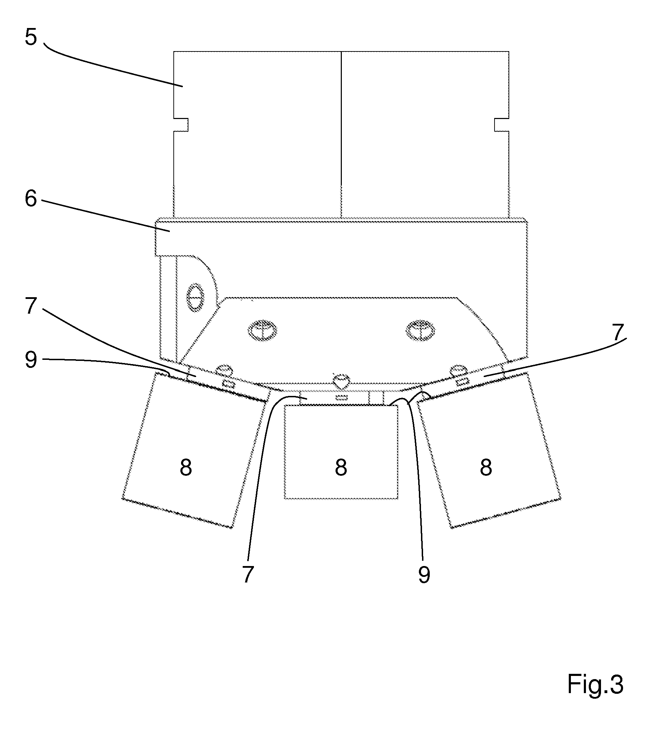

Die Lichterzeugungsmittel einschließlich der Lichtleitkanäle 8 sind in Fig.3 noch etwas detaillierter dargestellt. Die Lichtleitkanäle 8 sind im vorliegenden Beispiel als vorzugsweise mit einer nach innen reflektierenden Schicht versehene Glaskörper ausgestaltet, sie können aber auch als Hohlräume konzipiert werden. Hier sind drei Arrays 7 von lichtemittierenden Hochleistungsdioden dargestellt, die Abstrahlfläche jedes der Arrays 7 ist zu den Abstrahlflächen der jeweils anderen Arrays 7um einen in bezug auf das jeweils andere Array 7 spezifischen Kippwinkel gekippt angeordnet. Durch die Einstellung des Kippwinkels im Zusammenspiel mit den Lichtverteilungsmitteln ergibt sich ein gewisser Spielraum bei der Einstellung der Lichtintensität auf der Lichtaustrittsfläche 11. Während für das mittlere Array 7 der Kippwinkel 0 ist und von diesem aufgrund der Ausgestaltung der Lichtverteilungsmittel im wesentlichen der Frontzahnbereich beleuchtet wird, sind die beiden äußeren Arrays 7 relativ zum zentralen Array 7 um einen vom Betrag her gleichen Kippwinkel gekippt anordnet. Ein stärkerer Kippwinkel würde aufgrund von Reflexionen an den Begrenzungsflächen zu einer verstärkten Beleuchtung des Frontzahnbereiches bis hin zu den Eckzähnen führen, bei flacheren Kippwinkeln wird das Licht wieder zu den Seiten des Lichtverteilungsraumes geleitet und somit auf die seitlich angeordneten Zähne gelenkt.

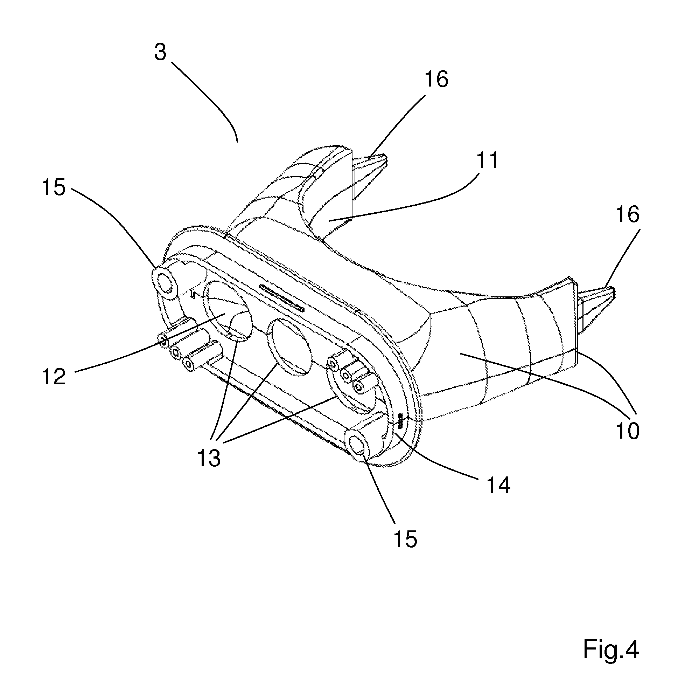

Über die Lichteintrittskanäle 8 wird das Licht in einen Lichtverteilungsraum im Aufsatzkörper 3 geleitet, der durch eine oder mehrere Begrenzungsflächen 10 sowie durch eine Lichtaustrittsfläche 11 begrenzt wird. Die Lichtaustrittsfläche 11 ist zu den Lichtleitkanälen 8 beabstandet angeordnet. Der Aufsatzkörper 3 mit den Lichtverteilungsmitteln ohne die Lichtleitkanäle 8 ist in Fig.4 detaillierter dargestellt. Er ist im wesentlichen als Hohlkörper ausgebildet, seine Wände bilden die Begrenzungsflächen 10 und die Lichtaustrittsfläche 11. Von der Lichtaustrittsfläche 11 wird das Licht auf die Zähne gelenkt. Sie weist eine Krümmung auf, die im wesentlichen der Krümmung des Gebisses folgt, so daß bei in den Mund eingebrachter Vorrichtung die Lichtaustrittsfläche 11 zu den zu bestrahlenden Zähnen im Mittel den gleichen Abstand aufweist. Die Begrenzungsflächen 10 und die Lichtaustrittsfläche 11 umschließen einen Hohlraum 12, der auf seiner rückwärtigen Seite noch Eintrittsöffnungen 13 für die Lichtleitkanäle 8 aufweist. Der Lichtverteilungsraum kann auch als mit einem für die Abstrahlwellenlängenbereiche transparenten Material ausgefüllt ausgestaltet sein, dies ist jedoch in der Fertigung aufwendiger. Auf ihrer Innenseite, also zum Lichtverteilungsraum weisend, sind die Begrenzungsflächen 10 zur Umlenkung von Licht auf die Lichtaustrittsfläche reflektierend ausgestaltet. Auf diese Weise wird dafür gesorgt, daß die von den Arrays 7 zur Verfügung gestellte Lichtleistung möglichst vollständig ausgenutzt werden kann.

Durch die Verwendung von Hochleistungsdioden mit einer optimierten Kühlung ist es möglich, die Vorrichtung so kompakt zu konzipieren, daß sie für die Zahnbleichung grundsätzlich in den Mund einer zu behandelnden Person eingeführt werden kann. Für eine höchstmögliche Effizienz ist es dabei vorteilhaft, wenn die Abstrahlwellenlängenbereiche der Hochleistungs-LEDs an den Absorptionswellenlängenbereich eines Farbstoffes, mit dem das Bleichgel versetzt ist, angepaßt ist oder umgekehrt, so daß sich beide Bereiche überlappen. Eine hohe Effizienz kann erreicht werden, wenn die Halbwertsbreite der Abstrahlwellenlängenbereiche bei etwa 30 nm bis 50 nm liegt, und ergänzend oder alternativ auch die zentralen Wellenlängen der Abstrahlwellenlängenbereiche im Bereich von 30 nm um eine Absorptionswellenlänge des im Bleichgel enthaltenen Farbstoffes, bei der die Absorption maximal ist, liegen. Aufgrund dieser effizienten Ausnutzung reicht eine relativ geringe Flächenleistungsdichte von 200 mW/cm² bereits aus, um den Farbstoff im Bleichgel zu aktivieren, wodurch das Bleichgel erwärmt wird und im Bleichgel enthaltenes Wasserstoffperoxid in Radikale gespalten wird. Diese setzen dann den Bleichungsprozeß in Gang, sobald der Farbstoff jedoch ausgeblichen ist, wird das Gel nicht weiter erwärmt, da die Flächenleistungsdichte dazu nicht ausreicht, insbesondere wenn der Abstrahlwellenbereich im grünen oder blauen Anteil des Farbspektrums gewählt wird. Der Bleichprozeß wird dann automatisch gestoppt.

Auf seiner rückwärtigen Seite weist der Aufsatzkörper 3 außerdem noch Mittel auf, mit denen er mit dem Grundkörper 1 verbunden werden kann. Diese umfassen einerseits einen Haltering 14, der beispielsweise aus einem harten Gummi hergestellt sein kann und in bezug auf eine entsprechende umlaufende Nut im Grundkörper 1 eine Überpassung aufweist, so daß eine kraftschlüssige Verbindung erreicht werden kann. Diese kann zusätzlich noch durch magnetische Kopplungen verstärkt werden, wobei Magnete beispielsweise im Grundköper 1 integriert sein können, und in entsprechenden Halterungen 15 magnetisierbare Metallelemente eingebracht sein können.

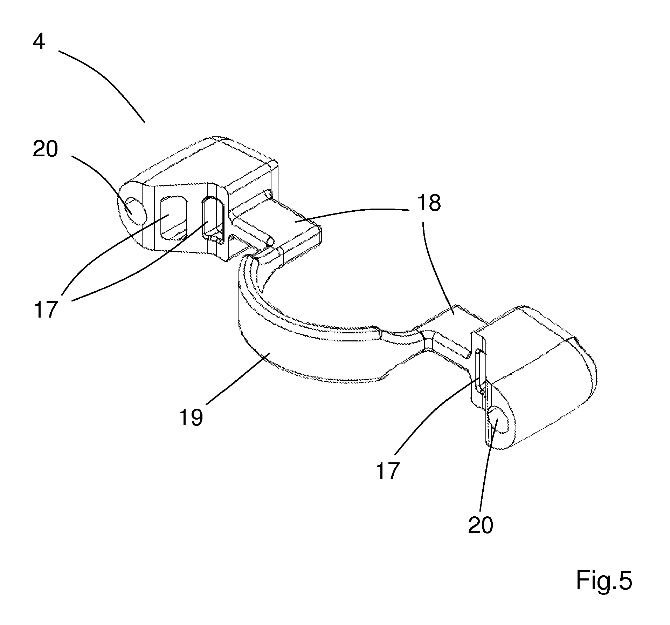

Auf der der zur behandelnden Person zugewandten Seite weist der Aufsatzkörper 3 außerdem noch Verbindungsstücke 16 auf, die in entsprechende Öffnungen 17 des Adapters 4 gesteckt werden können und den Aufsatzkörper kraftschlüssig mit dem Adapter 4 verbinden. Der Adapter 4 ist im Detail in Fig.5 dargestellt und wird bevorzugt ebenfalls aus einem elastischen Material hergestellt wird. Der Adapater 4 weist Adaptierungsmittel mit Beißauflagen 18 auf, auf die die Person während der Behandlung beißt, so daß eine vorgegebene Position der Vorrichtung im Mund dauerhaft beibehalten wird.

Der Adapter 4 verfügt auch über ein elastisches Gelenk 19, mit dem eine Anpassung an verschiedene Mundgrößen in einem begrenzten Rahmen möglich ist. Auf diese Weise ist es auch möglich, ein und denselben Adapter 4 für verschiedene Größen des Aufsatzkörpers 3 zu verwenden. Zu diesem Zweck weist der Adapter 4 auch mehrere nebeneinanderliegende Öffnungen 17 auf. Die Abmessungen sollten dabei aber nicht so groß sein, daß der Adapter im Mund als extrem störend empfunden wird.

Der Adapter 4 weist außerdem Anschlüsse 20 zum Anschluß von nicht gezeigten Absaugleitungen auf. Mit diesen Absaugleitungen kann während der Behandlung der natürlicherweise gebildete Speichel im Mund der Person abgesaugt werden.

Gegenüber den im Stand der Technik bekannten Vorrichtungen weist die vorangehend beschriebene Vorrichtung mehrere Vorteile auf. Aufgrund ihrer effizienten Lichtausnutzung und der effizienten Kühlung ist es möglich, sie im Mund des Patienten anzuordnen, was bisher nur bei solchen Vorrichtungen, die für den Heimgebrauch gedacht sind, möglich war, die jedoch eine wesentlich geringere Effizienz haben und bei denen die Behandlung nicht nur länger dauert, sondern in der Regel auch nicht so gute Ergebnisse zeigt. Die Vorrichtung arbeitet aufgrund der Wasserkühlung sehr leise, und aufgrund der modularen Bauweise läßt sich der Aufwand für den Wechsel des Aufsatzkörpers 3 bzw. des Adapters 4 in Grenzen halten.

Zur Durchführung der kosmetischen Bleichung von Zähnen wird in einem ersten Schritt ein Passivierungsgel auf das Zahnfleisch aufgetragen, um dieses vor dem Kontakt mit dem Bleichmittel zu schützen. Zusätzlich wird ein Wangen- und Lippenspreizer in den Mund eingebracht, so daß dieser während der Behandlung dauernd geöffnet bleibt und ein Verrutschen der Vorrichtung verhindert wird. Dann wird auf die zu behandelnden Zähne ein passives, Chromophore enthaltendes und photochemisch aktivierbares Bleichgel aufgebracht. Die Chromophore sind dabei so ausgelegt, daß sie Licht mit Wellenlängen aus einem vorgegebenen Absorptionswellenlängenbereich absorbieren.

Anschließend wird die Vorrichtung in den Mund eingebracht, wobei die zu behandelnde Person auf die Beißauflagen 18 des Adapters 4 beißt, auf diese Weise wird die Lichtaustrittsfläche 11 so fixiert, daß sie im wesentlichen und im Mittel den gleichen Abstand zu den zu behandelnden Zähnen aufweist. Da die Lichtaustrittsfläche 11 der Krümmung des Gebisses folgt und in den Mund eingebracht ist, werden auch die seitlichen Zähne direkt mit Licht bestrahlt, was bisher im Stand der Technik mittels der professionellen Zahnbleicheinrichtungen nicht möglich ist. Die zu behandelnden Zähne werden also gleichzeitig und im wesentlichen im gleichen Abstand zu einer Lichtaustrittsfläche, durch die von einer Lichtquelle abgestrahltes Licht austritt, beleuchtet. Als Lichtquelle werden die erwähnten Arrays vom lichtemittierenden Hochleistungsleuchtdioden verwendet, dabei sind die Abstrahlcharakteristiken der Leuchtdioden und die Absorptionscharakteristik der Chromophore so aneinander angepaßt, daß von den Hochleistungsleuchtdioden abgestrahlte Licht Wellenlängen aus einem Abstrahlwellenlängenbereich aufweist, der sich mit dem Absorptionswellenlängenbereich mindestens teilweise überlappt, so daß das Licht die Chromophore anregt. Die Chromophore wandeln die absorbierte Lichtenergie in Wärme um und spalten auf diese Weise im Bleichgel enthaltenes Wasserstoffperoxid auf. Die Spaltungsprodukte des Wasserstoffperoxids oxidieren im Zahn enthaltene Farbstoffe und bleichen den Zahn somit. Dabei achtet man auch darauf, die Abstrahlcharakteristik der Hochleistungsleuchtdioden möglichst schmal zu halten, mit einer Halbwertsbreite von nicht mehr als 100 nm, bevorzugt von 30 nm bis 50 nm, um eine möglichst vollständige Absorption des Lichtes durch die Chromophore zu erreichen. Auf diese Weise wird verhindert, daß die Zähne erwärmt werden, was für eine Folgebehandlung nachteilig wäre. Vorzugsweise, aber nicht ausschließlich, emittieren die Leuchtdioden im sichtbaren Spektralbereich von 400 nm bis 800 nm. In diesem Spektralbereich durchdringt das Licht teilweise den Zahn. Dadurch kann auch Bleichmittel, welches auf die Kaufflächen aufgetragen wurde und z.B. in die Fissuren eindringt, aktiviert werden. Die Behandlung, die in der Regel für alle Zähne innerhalb von 20 Minuten abgeschlossen werden kann, läuft dabei auch ohne die ständige Anwesenheit eines Behandlers ab. Die Bestrahlung wird automatisch beendet, wenn die Chromophore im Bleichgel ausgeblichen sind. Weiteres Licht wird dann nicht absorbiert. Dies kann beispielsweise über Sensoren registriert werden, so daß sich die Vorrichtung abschaltet und ein Signal ausgibt, welches an den Behandler weitergeleitet werden kann. Dieses Signal kann beispielsweise akustisch oder optisch sein.

Optional kann dann nach einer folgenden Reinigung ein Bleichgel mit einer gegenüber dem zuerst verwendeten Bleichgel niedrigeren Konzentration von Wasserstoffperoxid sowie einen Fluoranteil auf die Zähne aufgebracht werden, so daß die Zähne bei einer weiteren Bestrahlung versiegelt werden.

### Bezuaszeichenliste

- 1: Handstück
- 2: Schlauch
- 3: Aufsatzkörper
- 4: Adapter
- 5: Wärmetauscher
- 6: LED-Kühlung
- 7: Array mit Hochleistungsleuchtdioden
- 8: Lichtleitkanäle
- 9: Lichteintrittsfläche
- 10: Begrenzungsfläche
- 11: Lichtaustrittsfläche
- 12: Hohlraum
- 13: Eintrittsöffnung
- 14: Haltering
- 15: Halterungen
- 16: Verbindungsstück
- 17: Öffnung
- 18: Beißauflage
- 19: Gelenk
- 20: Anschluß

## Patentansprüche

1. Vorrichtung, mit der Zähne im Mund einer Person bei der Zahnbleichung mittels eines auf die Zähne aufgetragenen, photochemisch aktivierbaren Bleichgels bestrahlt werden, umfassend
- Lichterzeugungsmittel zur Erzeugung von Licht zur Bestrahlung der Zähne,
- Kühlmittel zur Kühlung der Lichterzeugungsmittel,
- Mittel zur Steuerung der auf die Zähne gerichteten Lichtdosis,
- Lichtverteilungsmittel, durch die das von den Lichterzeugungsmitteln erzeugte Licht in einem definierten Bereich zur gleichzeitigen Bestrahlung einer Vielzahl der Zähne abstrahlbar ist,
- Adaptierungsmittel zur Positionierung der Vorrichtung relativ zu den Zähnen,
- **dadurch gekennzeichnet, daß**
- die Lichterzeugungsmittel mindestens ein Array (7) von in einer Abstrahlfläche angeordneten, Licht emittierenden Hochleistungsdioden umfassen, wobei die Hochleistungsdioden zur Abstrahlung von Licht in Abstrahlwellenlängenbereichen mit einer Halbwertsbreite von jeweils bis zu 100 nm ausgestaltet sind und sich die Abstrahlwellenlängenbereiche mit einem Absorptionswellenlängenbereich mit Wellenlängen, in denen das Bleichgel aktivierbar ist, überlappen, und
- die Lichtverteilungsmittel eine Lichtaustrittsfläche (11) umfassen, von der das Licht auf die Zähne gelenkt wird, und welche eine Krümmung aufweist, die im wesentlichen der Krümmung des Gebisses folgt, so daß bei in den Mund eingebrachter Vorrichtung die Lichtaustrittsfläche (11) zu den zu bestrahlenden Zähnen im Mittel den gleichen Abstand aufweist.

2. Vorrichtung nach Anspruch 1, **dadurch gekennzeichnet, daß** die Halbwertsbreite der Abstrahlwellenlängenbereiche bei 30 nm bis 50 nm liegt und/oder die Zentralwellenlänge der Abstrahlwellenlängenbereiche in einem Bereich von 30 nm um die Wellenlänge liegt, die zu einer maximalen Aktivierung des Bleichmittels führt.

3. Vorrichtung nach Anspruch 1 oder 2, **dadurch gekennzeichnet, daß** die Lichterzeugungsmittel drei Arrays (7) von lichtemittierenden Hochleistungsdioden umfassen, wobei die Abstrahlfläche jedes der Arrays zu den Abstrahlflächen der jeweils anderen Arrays um einen in bezug auf das jeweils andere Array spezifischen Kippwinkel gekippt angeordnet ist.

4. Vorrichtung nach einem der vorgenannten Ansprüche, **dadurch gekennzeichnet, daß** die Hochleistungsdioden im Wellenlängenbereich von 400 nm bis 800 nm emittieren.

5. Vorrichtung nach einem der vorgenannten Ansprüche, **dadurch gekennzeichnet, daß** gleichzeitig mehrere Zähne einer oder beider Zahnreihen zur Bestrahlung vorgesehen sind, wobei die bestrahlte Fläche 5 cm² bis 40 cm², vorzugsweise 15 cm² bis 20 cm² beträgt.

6. Vorrichtung nach einem der vorgenannten Ansprüche, **dadurch gekennzeichnet, daß** die Lichtverteilungsmittel umfassen:
- entsprechend der Anzahl von Arrays (7) Lichtleitkanäle (8) mit Lichteintrittsflächen (9), die bevorzugt parallel zu den Abstrahlflächen angeordnet sind, und die Durchmesser in der Größenordnung der Arrays aufweisen, und
- einen an die Lichtleitkanäle anschließenden Lichtverteilungsraum, der durch eine oder mehrere Begrenzungsflächen (10) sowie die Lichtaustrittsfläche (11), die zu den Lichtleitkanälen (8) beabstandet angeordnet ist, begrenzt wird.

7. Vorrichtung nach Anspruch 6, **dadurch gekennzeichnet, daß** die Begrenzungsflächen (10) zwecks Umlenkung von Licht auf die Lichtaustrittsfläche (11) reflektierend ausgestaltet sind, und/oder daß der Lichtverteilungsraum als Hohlraum (12) oder als mit einem für die Abstrahlwellenlängenbereiche transparenten Material ausgefüllt ausgestaltet ist.

8. Vorrichtung nach Anspruch 6 oder 7, **dadurch gekennzeichnet, daß** die Begrenzungsflächen (10) zwecks Umlenkung von Licht auf die Lichtaustrittsfläche (11) so ausgestaltet sind, daß von den Zahnoberflächen reflektiertes Licht vollständig oder teilweise wieder in Richtung Zahnoberfläche zurück gelenkt werden.

9. Vorrichtung nach einem der vorgenannten Ansprüche, **dadurch gekennzeichnet, daß** die Kühlungsmittel eine Wasserkühlung mit mindestens einer Zu- und mindestens einer Ableitung umfassen.

10. Vorrichtung nach einem der vorgenannten Ansprüche, **dadurch gekennzeichnet, daß** die Adaptierungsmittel Beißauflagen (18) aufweisen, auf die die Person während der Behandlung beißt, so daß eine vorgegebene Position der Vorrichtung im Mund für die Dauer der Behandlung beibehalten wird.

11. Vorrichtung nach einem der vorgenannten Ansprüche, **dadurch gekennzeichnet, daß** die Adaptierungsmittel Anschlüsse (20) zum Anschluß von Absaugleitungen zur Absaugung von Speichel aufweisen.

12. Vorrichtung nach einem der vorgenannten Ansprüche, **dadurch gekennzeichnet, daß** die Lichtleistung im zeitlichen Mittel über eine Pulsweitenmodulation variabel einstellbar ist, um **dadurch** die Lichtdosis anzupassen.

13. Vorrichtung nach einem der vorgenannten Ansprüche, **dadurch gekennzeichnet, daß** die Lichtdosis durch die Variation der Bestrahlungsdauer einstellbar ist.

14. Vorrichtung nach einem der vorgenannten Ansprüche, **dadurch gekennzeichnet, daß** sie modular aus von einem Bediener ohne mechanische Hilfsmittel separier- und zusammenfügbaren Modulen zusammengesetzt ist, umfassend (i) einen als Handstück (1) ausgestalteten Grundkörper, umfassend die Lichterzeugungsmittel und die Kühlungsmittel, (ii) einen als Aufsatz ausgestalteten Aufsatzkörper (3), umfassend mindestens einen Teil der Lichtverteilungsmittel, und (iii) einen Adapter (4), umfassend die Adaptierungsmittel.

15. Verfahren zur kosmetischen Bleichung von Zähnen, bei dem
- auf die zu behandelnden Zähne ein passives, Chromophore enthaltendes und photochemisch aktivierbares Bleichgel aufgebracht wird, wobei die Chromophore Licht mit Wellenlängen aus einem Absorptionswellenlängenbereich absorbieren,
- die zu behandelnden Zähne gleichzeitig und im wesentlichen im gleichen Abstand zu einer Lichtaustrittsfläche, durch die von einer Lichtquelle abgestrahltes Licht austritt, beleuchtet werden, wobei das Licht Wellenlängen aus einem Abstrahlwellenlängenbereich aufweist, dessen Halbwertsbreite nicht größer als 100 nm ist, und der sich mit dem Absorptionswellenlängenbereich mindestens teilweise überlappt,
- so daß das Licht die Chromophore anregt, welche die absorbierte Energie in Wärme umwandeln und auf diese Weise im Bleichgel enthaltenes H₂O₂ aufspaltet, dessen Spaltungsprodukte im Zahn enthaltene Farbstoffe oxidieren und den Zahn bleichen.

16. Verfahren nach Anspruch 15, **dadurch gekennzeichnet, daß** vor dem Auftragen des Bleichgels ein Passivierungsgel auf das Zahnfleisch aufgetragen wird und/oder ein Wangen- und Lippenspreizer in den Mund zu dessen dauernder Öffnung während der Behandlung eingebracht wird.

17. Verfahren nach Anspruch 15 oder 16, **dadurch gekennzeichnet, daß** im Anschluß an die Bestrahlung und eine Reinigung ein weiteres Bleichgel mit einer gegenüber dem zuerst verwendeten Bleichgel niedrigeren Konzentration von H₂O₂ sowie einem Fluoranteil auf die Zähne aufgebracht wird, so daß die Zähne bei einer weiteren Bestrahlung versiegelt werden.

18. Verfahren nach einem der Ansprüche 15 bis 17, **dadurch gekennzeichnet, daß** die Bestrahlung automatisch beendet wird, wenn die Chromophore im Bleichgel ausgeblichen sind.

19. Verfahren nach einem der Ansprüche 15 bis 18, **dadurch gekennzeichnet, daß** die Aktivierung des Bleichmittels automatisch in Abhängigkeit davon beendet wird, daß die Chromophore im Bleichgel ausgeblichen sind.
